# EUROPEAN PATENT APPLICATION

(11) **EP 1 746 419 A1**
(43) Date of publication of application: **24.01.2007**
(21) Application number: 05737339.1
(22) Date of filing: 28.04.2005
(51) Int. Cl.: G01N 33/48

(54) **SAMPLE ANALYZING TOOL**

(30) Priority: 30.04.2004 JP 2004136062
(71) Applicant: Arkray, Inc., Kyoto-shi, Kyoto 601-8045 (JP)
(72) Inventor: IKETANI, Kazuya, c/o Arkray, Inc., Kyoto-shi, Kyoto 601-8045 (JP)
(74) Representative: Neath, Susannah Mairi
(86) International application number: PCT/JP2005/008176
(87) International publication number: WO 2005/106463

(57) **Abstract**

A sample analysis tool that has a simple structure and is capable of carrying out a blood cell separation of high hematocrit blood with a high separation ability is provided. The sample analysis tool according to the present invention includes a blood cell separation layer including a lateral flow filtration filter layer. For example, a sample analysis tool 1 in which a reagent layer 17 is formed on a substrate 11. The blood cell separation layer is stacked on the reagent layer 17. A part or a whole of an upper surface of the blood cell separation layer serves as a blood sample supplying portion. A part or a whole of a lower surface of the reagent layer 17 serves as a measuring portion. The blood sample supplied from the supplying portion is separated into blood cells and plasma by the blood cell separation layer. The plasma is introduced into the reagent layer 17, and a component in the plasma is measured in the measuring portion is formed to have a structure in which the blood cell separation layer includes a lateral flow filtration filter layer 13a. The blood sample introduced into the lateral flow filtration filter layer 13a is subjected to a blood cell separation while moving in a lateral flow direction.

## Description

### Technical Field

The present invention relates to a sample analysis tool.

### Background Art

In biochemical tests and clinical tests, a sample analysis tool (also referred to as a test strip) conventionally has been used in various applications. The sample analysis tool generally has a configuration in which a reagent layer formed of a filter paper or the like impregnated with a reagent is disposed on a plastic substrate. In the test analysis tool, a specimen such as urine or blood is brought into contact with the above-noted reagent layer so as to react with various reagents, thereby conducting a qualitative analysis or a quantitative analysis by chromogenic reaction, change in color tone or the like. This analysis is carried out by visual observation in some cases and using a measuring apparatus in other cases. Recently, a fully automatic measuring apparatus that automatically handles steps from sampling of a specimen to measuring thereof also has been developed and commercialized. In the case of analyzing a component in plasma such as a glucose concentration in blood, it is necessary to separate blood cells from the blood and allow the plasma alone to react with a reagent. Accordingly, in a sample analysis tool for blood tests, a blood cell separation layer such as a glass filter is disposed on the reagent layer. Also, in a usual sample analysis tool, a cover is disposed on the blood cell separation layer via a spacer. This cover is provided with an opening for supplying a blood sample. When blood is made to drop through this opening and adhere onto an upper surface of the blood cell separation layer, blood cells are trapped by a filter effect while the blood is passing through the blood cell separation layer in a thickness direction (in a vertical flow direction), and the plasma alone moves to the reagent layer, where the component in the plasma and the reagent react with each other. At the position of the substrate corresponding to the reagent layer, an opening for measurement usually is provided, from which chromogenic reaction or a change in color tone is measured. In this case, if the blood cell separation is not successful, the color of erythrocytes may inhibit the measurement. Especially when a hematocrit value (Ht) is high (for example, equal to or higher than Ht 60%), the blood cell separation may be carried out unsuccessfully, thus affecting the analysis. Further, in blood analyses, for minimizing the physical pain of a test subject, the reduction of the amount of blood necessary for the analysis has been an issue to be tackled. In order to solve these problems, various attempts have been made conventionally.

For instance, there is a technology in which a layered product of a plurality of glass filters is compressed, thus improving a blood cell separation speed and a blood cell separation ability and reducing the necessary amount of blood (for example, see Patent documents 1 to 6). In this case, for example, the layered glass filters are compressed so as to have a thickness equal to or smaller than 75% of their original thickness or an area 14% to 43% of their original area. Such a technology is said to allow an efficient blood cell separation of even a slight amount (e.g., 5 to 10 µl) of blood. However, in order to maintain the compressed state of the glass filters, the sample analysis tool has to have a special structure, which causes problems of a reduced efficiency and an increased cost in manufacturing the sample analysis tool.

There also is a technology in which a glass filter is impregnated with a medicine such as a blood cell separation agent (a mixed agent of NaCl and sorbitol, an amino acid or the like) or an anticoagulant, thus improving the blood cell separation efficiency (for example, see Patent documents 7 to 10). However, this technology requires a medicine impregnation step, which causes problems of a reduced efficiency and an increased cost in manufacturing the sample analysis tool.

Further, there also is a technology in which an asymmetrical porous membrane is utilized for the blood cell separation layer (for example, see Patent document 11). However, the asymmetrical porous membrane is a special element and thus costs a lot.
Patent document 1: US Patent 5139685
Patent document 2: WO 09/319831 A
Patent document 3: EP Patent 0633808
Patent document 4: US Patent 6096269
Patent document 5: EP Patent 0640392
Patent document 6: JP Patent 3439836
Patent document 7: WO 2003/056163 A
Patent document 8: JP 09(1997)-196910 A
Patent document 9: JP 09(1997)-196908 A
Patent document 10: JP 10(1998)-185909 A
Patent document 11: EP 0407800 A

### Disclosure of Invention

### Problem to be Solved by the Invention

With the foregoing in mind, it is an object of the present invention to provide a sample analysis tool that has a simple structure, is low in cost and has an excellent blood cell separation ability.

### Means for Solving Problem

In order to achieve the above-mentioned object, a sample analysis tool according to the present invention includes a blood cell separation layer, a reagent, and a measuring portion. The blood cell separation layer includes a lateral flow filtration filter layer. The measuring portion is located in a lower part of the blood cell separation layer. A supplied blood sample is subjected to a blood cell separation while moving in the lateral flow filtration filter layer in a lateral flow direction. The blood sample that has been subjected to the blood cell separation reacts with the reagent, and a component in the blood sample subjected to the blood cell separation is measured in the measuring portion.

### Effects of the Invention

As described above, the sample analysis tool according to the present invention is characterized by using the blood cell separation layer (the blood cell separation filter) including the lateral flow filtration filter layer. In other words, although it is necessary for improving a blood cell separation ability to increase the moving distance of a blood sample in a blood cell separation layer, a conventional filter has had a limitation in extending the blood moving distance because the blood sample moves in a vertical flow direction and its moving distance is defined by the filter thickness. In contrast, the blood cell separation layer including the lateral flow filtration filter layer is used in the present invention, so that the blood sample is subjected to a blood cell separation while moving in the lateral flow filtration filter layer in a lateral flow direction. Consequently, it becomes possible to extend the moving distance of the blood without increasing the filter thickness. In addition, according to the present invention, the blood sample easily spreads in a lateral flow direction. Therefore, the color develops more uniformly in a measuring portion, and the time needed for measurement is reduced further. Also, since the blood cell separation layer in the present invention has a simple structure, the manufacture of the sample analysis tool of the present invention does not become complicated and the cost thereof does not increase. The present invention allows a blood cell separation with a high separation ability even for a blood sample with a high hematocrit, for example. As a result, it becomes possible to conduct a highly accurate and reliable blood test or the like.

In the present invention, the "vertical flow direction" refers to a thickness direction of the blood cell separation layer, whereas the "lateral flow direction" refers to a direction perpendicular to the thickness direction of the blood cell separation layer.

In the present invention, the "lateral flow filtration filter layer" means a filter layer whose principal filtration direction is the lateral flow direction. In the "lateral flow filtration filter layer", a blood sample also moves in the vertical flow direction in some cases. Further, in the present invention, the "vertical flow filtration filter layer" means a filter layer whose principal filtration direction is the vertical flow direction. In the "vertical flow filtration filter layer", a part of a blood sample also moves in the lateral flow direction in some cases.

In the present invention, the "sample analysis tool" means the same thing as a "test strip".

In the present invention, a "blood sample subjected to a blood cell separation" is plasma or serum, for example.

### Brief Description of Drawings

[FIG. 1] FIG. 1 is a perspective view showing an example of a sample analysis tool of the present invention.
[FIG. 2] FIG. 2 is a sectional view taken along an A-A direction showing the sample analysis tool.
[FIG. 3] FIG. 3 is a sectional view taken along a B-B direction showing the sample analysis tool.
[FIG. 4] FIG. 4 is a sectional view showing an example of a state in which a blood sample moves.
[FIG. 5] FIG. 5 is a perspective view showing another example of the sample analysis tool of the present invention.
[FIG. 6] FIG. 6 is a sectional view taken along an A-A direction showing the sample analysis tool.
[FIG. 7] FIG. 7 is a sectional view taken along a B-B direction showing the sample analysis tool.
[FIG. 8] FIG. 8 is a sectional view showing another example of the state in which the blood sample moves.
[FIG. 9] FIG. 9 is a sectional view showing yet another example of the sample analysis tool of the present invention.
[FIG. 10] FIG. 10 is a sectional view showing yet another example of the sample analysis tool of the present invention.
[FIG. 11] FIG. 11 is a plan view showing yet another example of the sample analysis tool of the present invention.
[FIG. 12] FIG. 12 is a plan view showing yet another example of the sample analysis tool of the present invention.
[FIG. 13] FIG. 13 is a graph regarding the blood cell separation ability in yet another example of the sample analysis tool of the present invention.

### Explanation of reference numerals

- 1: Sample analysis tool

- 11: Substrate
- 12: Cover layer
- 13: Blood cell separation layer
- 13a: Lateral flow filtration filter layer
- 13b: Vertical flow filtration filter layer
- 14: Spacer layer
- 15: Opening for supplying blood sample
- 16: Opening for measurement
- 17: Reagent layer
- 18: Spreading layer
- 19: Blood sample supplying portion
- 20: Water-repellent portion
- 121: Lid

### Description of the Invention

In the sample analysis tool of the present invention, there is no particular limitation on where the reagent is disposed. The reagent may be disposed in a part of the blood cell separation layer or in a reagent layer that is prepared in a separate process as described later. In the case where the reagent is disposed in a part of the blood cell separation layer, it is preferable that the reagent is disposed on a side opposite to a blood supply side. For example, when an upper part of the blood cell separation layer serves as a blood sample supplying portion, it is preferable that the reagent is disposed in a lower part of the blood cell separation layer.

In the sample analysis tool of the present invention, the supplied blood sample may react with the reagent while being subjected to a blood cell separation or react with the reagent after the blood cell separation.

In the sample analysis tool of the present invention, the measuring portion is not particularly limited as long as it is located in a lower part of the blood cell separation layer. For example, the measuring portion may be a part or a whole of a lower surface of the blood cell separation layer or a part or a whole of a lower surface of a reagent layer that is provided below the blood cell separation layer in a separate process as described later.

In the sample analysis tool of the present invention, it is preferable to have a supplying system for supplying the blood sample to a portion in the lateral flow filtration filter layer away from a portion corresponding to a center of the measuring portion. With such a configuration, the moving distance of the blood sample can be extended further, making it possible to carry out a blood cell separation more efficiently. Accordingly, plasma or serum after the separation concentrates in the center of the measuring portion, which is advantageous in the measurement.

In the sample analysis tool of the present invention, it is preferable further to include a substrate, and a reagent layer. It is preferable that the reagent layer includes the reagent, the reagent layer is disposed on the substrate. The blood cell separation layer is stacked on the reagent layer.
A part or a whole of an upper surface of the blood cell separation layer serves as a blood sample supplying portion. A part or a whole of a lower surface of the reagent layer serves as the measuring portion. The blood sample supplied from the supplying portion is subjected to the blood cell separation by the blood cell separation layer. The blood sample that has been subjected to the blood cell separation is introduced into the reagent layer and reacts with the reagent, and the component in the blood sample subjected to the blood cell separation is measured in the measuring portion.

In the sample analysis tool of the present invention, it is preferable that the blood cell separation layer further includes a vertical flow filtration filter layer. The vertical flow filtration filter layer is stacked on the lateral flow filtration filter layer, and the supplied blood sample first is subjected to a blood cell separation while moving in the vertical flow filtration filter layer in a vertical flow direction and then subjected to the blood cell separation while moving in the lateral flow filtration filter layer in the lateral flow direction. With such a configuration, the moving distance of the blood sample can be extended further, making it possible both to carry out a blood cell separation efficiently and to miniaturize the entire sample analysis tool.

In the sample analysis tool of the present invention, it is preferable to have a configuration in which a cover layer further is disposed on the blood cell separation layer, the cover layer has an opening for supplying the blood sample to the blood cell separation layer, and a portion of an upper surface of the blood cell separation layer corresponding to the opening serves as a blood sample supplying portion.

In the sample analysis tool of the present invention, it is preferable to have a configuration in which a spacer layer further is disposed on the substrate, and the cover layer is disposed on the spacer layer and the blood cell separation layer. In this case, it is preferable that the spacer layer adheres to both the substrate and the cover layer and has a thickness designed to be smaller than a pre-compressed thickness of the blood cell separation layer, whereby the blood cell separation layer is compressed by the cover layer except the opening. This is because, in this way, it becomes possible to produce the same effect as supplying the blood sample first to the portion in the lateral flow filtration filter layer away from the portion corresponding to the center of the measuring portion, so that the moving distance of the blood sample can be extended further as described later. Also, in this way, the blood sample is dropped to the lateral flow filtration filter layer in the shape of a circle corresponding to the opening in the cover layer. The above-noted shape of a circle is not particularly limited and can be, for example, a ring shape, a polygonal shape or the like.

The degree to which the blood cell separation layer is compressed is such that a compressed thickness is, for example, at least 30 and smaller than 100, preferably 40 to 70 and more preferably 40 to 55, where the pre-compressed thickness of the blood cell separation layer is expressed by 100. Incidentally, although there is a conventional technology of compressing a glass filter so as to improve a blood cell separation ability as described earlier, the purpose and effect of compression of this technology are different from those of the present invention.

In the present invention, the degree to which the blood cell separation layer is compressed refers to the degree to which a compressed portion is compressed and does not include the degree with respect to a portion in the blood cell separation layer corresponding to the opening in the cover layer, for example, because this portion is not compressed. Also, in the case of compressing the reagent layer and the blood cell separation layer, the degree to which the reagent layer is compressed is not particularly limited and is such that a compressed thickness is, for example, at least 30 and smaller than 100, preferably 40 to 70 and more preferably 40 to 55, where the pre-compressed thickness of the reagent layer is expressed by 100.

In the sample analysis tool of the present invention, it is preferable to have a configuration in which a spreading layer further is stacked on the blood cell separation layer. The spreading layer can be formed of a mesh, a non-woven fabric, a filter paper or the like, for example.

In the sample analysis tool of the present invention, it is preferable to have a configuration in which the substrate has an opening for measurement at a position matched with the measuring portion of the reagent layer.

In the sample analysis tool of the present invention, it is preferable that the opening in the cover layer has a larger diameter than the opening for measurement in the substrate.

In the sample analysis tool of the present invention, it is preferable that the blood sample supplying portion on the upper surface of the blood cell separation layer except an end portion thereof is treated to be water repellent. In this way, the blood sample is supplied in the shape of a circle to the portion in the lateral flow filtration filter layer away from the portion corresponding to the center of the measuring portion. Consequently, the moving distance of the blood sample can be extended further. The above-noted shape of a circle is not particularly limited and can be, for example, a ring shape, a polygonal shape or the like.

In the sample analysis tool of the present invention, it is preferable that the opening in the cover layer is substantially circular. In this way, the blood sample is supplied in the shape of a circle to the portion in the lateral flow filtration filter layer away from the portion corresponding to the center of the measuring portion. Consequently, the moving distance of the blood sample can be extended further. The above-noted shape of a circle is not particularly limited and can be, for example, a ring shape, a polygonal shape or the like.

In the sample analysis tool of the present invention, an item to be tested can be, for example, glucose, cholesterol, triglyceride, ammonia, a uric acid, creatinine, urea nitrogen, calcium, inorganic phosphorus, magnesium, GGT, GOT, GPT, LDH, amylase, a total protein amount, albumin, fructosamine, creatine phosphokinase, bilirubin, alkaline phosphatase, HDL or the like.

In the sample analysis tool of the present invention, it is preferable that the vertical flow filtration filter layer and the lateral flow filtration filter layer are formed of a glass filter.

Now, examples of the sample analysis tool of the present invention will be described. It should be noted that the present invention is not limited to the examples below.

### Example 1

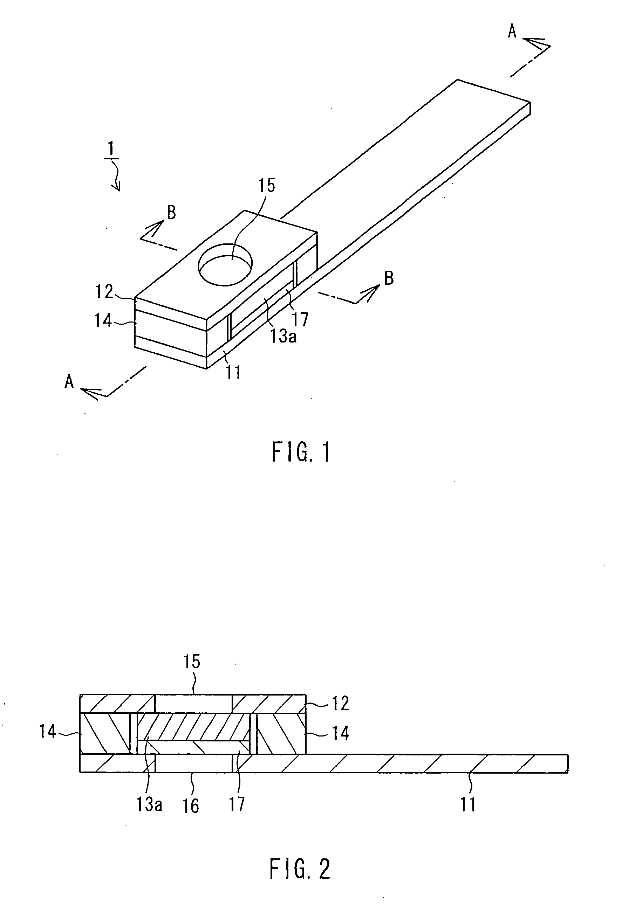

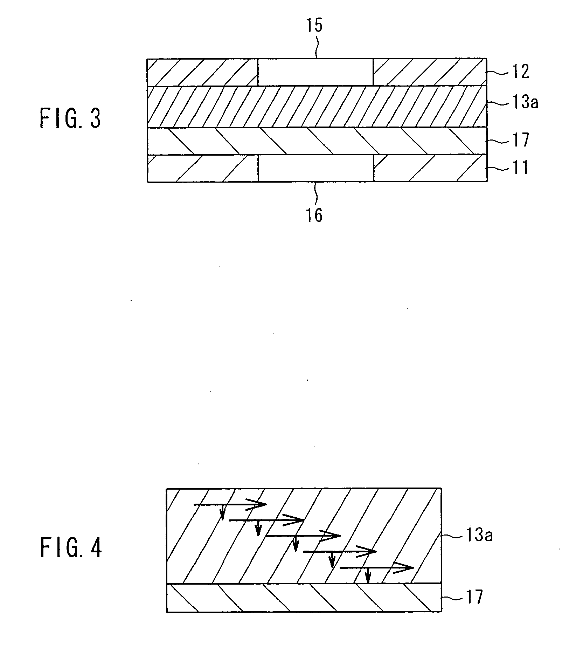

FIGs. 1, 2 and 3 illustrate an example of a sample analysis tool according to the present invention. FIG. 1 is a perspective view showing the above-noted sample analysis tool, FIG. 2 is a sectional view taken along an A-A direction in FIG. 1, and FIG. 3 is a sectional view taken along a B-B direction in FIG. 1. In these figures, the same portions are given the same reference numerals.

As shown in the figures, this sample analysis tool 1 has the following configuration. Two spacer layers 14 are disposed on a substrate 11, and a reagent layer 17 is disposed between these spacer layers 14. A blood cell separation layer formed of a lateral flow filtration filter layer 13a is disposed on the reagent layer 17, and a cover layer 12 further is disposed thereon so as to cover the spacer layers 14 and the lateral flow filtration filter layer 13a.
In substantially the central portion of the cover layer 12, a circular opening 15 is formed for supplying a blood sample. An upper surface of the blood cell separation layer corresponding to this opening 15 serves as a blood sample supplying portion. Also, a circular opening 16 for measurement is formed in the substrate 11 so as to be located at a position matched with the circular opening 15. A lower surface of the reagent layer 17 corresponding to this opening 16 serves as a measuring portion. Incidentally, in this example, the filter layer 13a may have a single layer structure or a structure in which a plurality of lateral flow filtration filter layers are stacked.

The dimensions of the substrate 11 are not particularly limited. In the case where the substrate 11 has a strip shape, its dimensions are, for example, 20 to 100 mm long by 3 to 10 mm wide by 0.1 to 0.7 mm thick, preferably 30 to 80 mm long by 4 to 8 mm wide by 0.1 to 0.5 mm thick, and more preferably 40 to 60 mm long by 5 to 7 mm wide by 0.1 to 0.3 mm thick. The material of the substrate is not particularly limited, either, and can be, for example, polyethylene terephthalate (PET), polyethylene (PE), polypropylene (PP), polymethyl methacrylate (PMMA), polyvinyl chloride (PVC), polycarbonate (PC) or the like. Among them, polyethylene terephthalate (PET) and polycarbonate (PC) are preferable, and polyethylene terephthalate (PET) is particularly preferable. Moreover, although the opening 16 for measurement formed in the substrate 11 is circular in these figures, the present invention is not limited to this. For example, the opening 16 may be rectangular. Furthermore, the dimensions of the opening 16 are not particularly limited, either. In the case where the opening 16 has a circular shape, it has a diameter ranging, for example, from 3 to 6 mm, preferably from 3.5 to 5.5 mm and more preferably from 4.3 to 4.8 mm. The opening 16 can be formed by a laser processing or a perforating treatment using a dedicated tool, for example.

The reagent layer 17 is prepared by impregnating a porous member with a reagent, followed by drying, for example. The above-noted porous member can be, for example, a non-fibrous porous member or a fibrous porous member. In particular, it is preferable to use a non-fibrous porous member. The non-fibrous porous member can be a porous member made of, for example, 6-nylon, 6,6-nylon, cellulose acetate, cellulose nitrate, polyethylene, polypropylene or the like. The fibrous porous member can be, for example, a filter paper, a non-woven fabric, a woven fabric or the like. Among them, a porous member made of 6-nylon, 6,6-nylon, cellulose acetate or cellulose nitrate is more preferable, and a porous member made of 6-nylon or 6,6-nylon is particularly preferable. The dimensions of the reagent layer 17 are not particularly limited. In the case where the reagent layer 17 has a rectangular planar shape, its dimensions are in the range of, for example, 3 to 10 mm long by 3 to 10 mm wide by 0.1 to 0.5 mm thick, preferably 4 to 8 mm long by 4 to 8 mm wide by 0.1 to 0.3 mm thick, and more preferably 5 to 7 mm long by 5 to 7 mm wide by 0.1 to 0.2 mm thick. Further, although the planar shape of the reagent layer 17 shown in these figures is rectangular, the present invention is not limited to this. Other than the above, the reagent layer 17 may have a circular planar shape, for example. The kind of the reagent for impregnation is determined suitably according to test items. The reagent used in the case of measuring glucose can be, for example, glucose oxidase, peroxidase, a surfactant, a binder, a dye or the like. The reagent used in the case of measuring cholesterol can be, for example, cholesterol oxidase, cholesterol esterase, peroxidase, a surfactant, a binder, a dye or the like. The reagent layer 17 can be produced by, for example, preparing a necessary reagent solution of various kinds and impregnating a porous member with this solution, followed by drying (for example, natural drying, air drying, heated-air drying or the like).

The lateral flow filtration filter layer 13a is not particularly limited as long as it has a function of moving (or spreading) a liquid in the lateral flow direction by a capillary action. For example, a glass filter, a polysulfone membrane, a polyethersulfone membrane, a nitrocellulose membrane or the like can be used. Among them, a glass filter and a polyethersulfone membrane are preferable, and a glass filter is more preferable. Such a lateral flow filtration glass filter may be a commercially available product. The dimensions of the lateral flow filtration filter layer 13a are not particularly limited. In the case where the lateral flow filtration filter layer 13a has a rectangular planar shape, its dimensions are in the range of, for example, 3 to 10 mm long by 3 to 10 mm wide by 0.1 to 0.7 mm thick, preferably 4 to 8 mm long by 4 to 8 mm wide by 0.1 to 0.5 mm thick, and more preferably 5 to 7 mm long by 5 to 7 mm wide by 0.2 to 0.3 mm thick. Although the planar shape of the lateral flow filtration filter layer 13a shown in these figures is rectangular, the present invention is not limited to this. The lateral flow filtration filter layer 13a may have a circular planar shape, for example. As described earlier, in the lateral flow filtration filter layer 13a in the present invention, although a blood cell separation is performed while a blood sample is moving principally in the lateral flow direction, there is no particular limitation to this. A part of the blood sample also moves in the vertical flow direction.

The material of the spacer layer 14 can be, for example, a foam or a rubber. The above-noted foam can be, for example, a polyethylene foam, a polyurethane foam, a polyolefin foam or the like. The above-noted rubber can be, for example, a chloroprene rubber, a butyl rubber, a urethane rubber, a butadiene rubber, a natural rubber, a silicone rubber, a styrene rubber or the like. Among them, a polyethylene foam, a polyurethane foam, a butyl rubber, a urethane rubber and a butadiene rubber are preferable, and a polyurethane foam and a butyl rubber are more preferable. The dimensions of the spacer layer 14 are not particularly limited. In the case where the spacer layer 14 has a rectangular planar shape, its dimensions are in the range of, for example, 3 to 10 mm long by 3 to 10 mm wide by 0.5 to 1.2 mm thick, preferably 4 to 8 mm long by 4 to 8 mm wide by 0.6 to 1.0 mm thick, and more preferably 5 to 7 mm long by 5 to 7 mm wide by 0.7 to 0.9 mm thick. Although the planar shape of the spacer layer 14 shown in the figures is rectangular, the present invention is not limited to this. The spacer layer 14 may have a circular planar shape, for example.

The material of the cover layer 12 can be, for example, polyethylene terephthalate (PET), polycarbonate (PC), polyethylene (PE), polypropylene (PP), polymethyl methacrylate (PMMA), polyvinyl chloride (PVC) or the like. Among them, polyethylene terephthalate (PET) and polycarbonate (PC) are preferable, and polyethylene terephthalate (PET) is more preferable. The dimensions of the cover layer 12 are not particularly limited. In the case where the cover layer 12 has a rectangular planar shape; its dimensions are in the range of, for example, 15 to 35 mm long by 3 to 10 mm wide by 0.1 to 0.4 mm thick, preferably 17 to 30 mm long by 4 to 8 mm wide by 0.1 to 0.3 mm thick, and more preferably 20 to 25 mm long by 5 to 7 mm wide by 0.1 to 0.2 mm thick. Although the planar shape of the cover layer 12 shown in the figures is rectangular, the present invention is not limited to this. The cover layer 12 may have a circular planar shape, for example. Moreover, although the opening 15 for supplying a blood sample formed in the cover layer 12 is circular in these figures, the present invention is not limited to this. For example, the opening 15 may be rectangular. Furthermore, the dimensions of the opening are not particularly limited, either. In the case where the opening has a circular shape, it has a diameter ranging, for example, from 4.8 to 8 mm, preferably from 4.8 to 7 mm and more preferably from 4.8 to 6 mm. The opening 15 can be formed by a laser processing or a perforating treatment using a dedicated tool, for example.

This sample analysis tool can be manufactured by stacking the above-mentioned various members and making them adhere to each other. The adhering method can be, for example, a method using an adhesive, a method using a double-coated adhesive tape, a laminating method by heat sealing, or the like. The above-noted adhesive can be, for example, an acrylic adhesive, an elastomeric adhesive or the like.

Now, this sample analysis tool is used as follows, for example. First, a fingertip is caused to bleed with a dedicated lancet or the like. This blood is supplied to the blood sample supplying portion through the opening 15 for supplying a blood sample in the cover layer 12. Incidentally, the blood supplying method is not limited to the above but can be a method in which a blood sample that has been prepared in advance is supplied using a capillary tube, an automatic pipette or the like, for example. FIG. 4 is a sectional view showing the state of blood cell separation, where the same portions as those in FIG. 1 are given the same reference numerals. As shown in the figure, the supplied blood sample first adheres to the upper surface of the lateral flow filtration filter layer 13a (the blood sample supplying portion) and infiltrates into the above-noted filter layer. Then, as indicated by arrows in the figure, the blood sample moves inside the filter layer 13a principally in the lateral flow direction, and at this time, a blood cell separation is conducted. Since the blood sample also moves in the vertical flow direction while moving in the lateral flow direction in the lateral flow filtration filter layer 13a, the plasma obtained by the blood cell separation is introduced into the reagent layer 17, where it reacts with the reagent that is provided in advance, so that a chromogenic reaction (a color change) or a change in color tone of the reagent occurs. Then, the chromogenic reaction or the change in color tone of the measuring portion is measured from the opening 16 for measurement in the substrate 11 of the sample analysis tool. This measurement may be carried out by visual observation or by using a general spectrophotometer, a dedicated measuring apparatus or the like. It should be noted that, although FIG. 4 schematically shows the movement of the blood sample and the arrows along the lateral flow direction indicate the rightward movement of the blood sample, the blood sample of course moves rightward at the same time and also moves downward. Accordingly, in the lateral flow filtration filter in the present invention, the blood sample gradually drops while moving in the lateral flow direction, so that its moving distance can be extended compared with the conventional case.

### Example 2

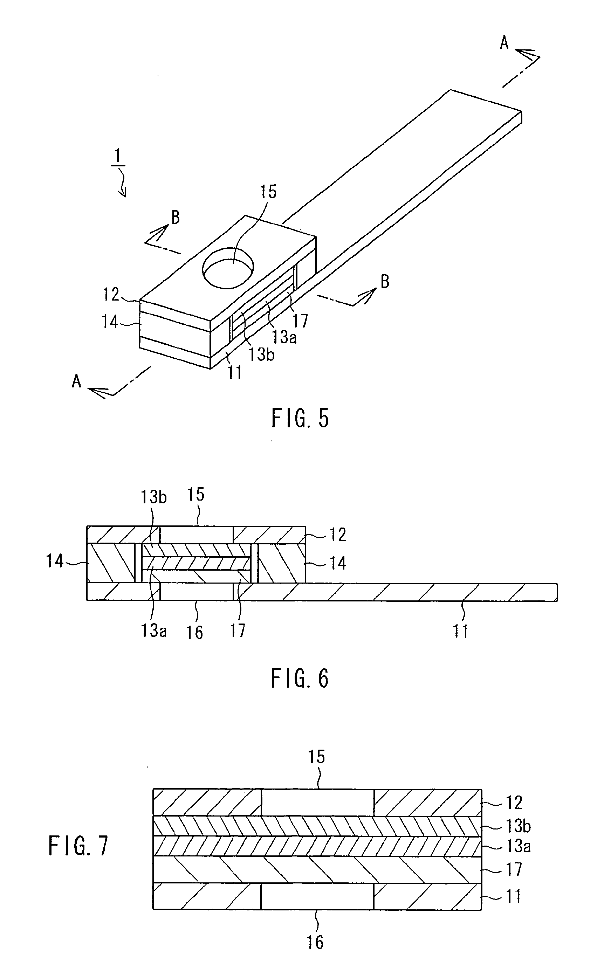

Next, FIGs. 5, 6 and 7 illustrate another example of the sample analysis tool according to the present invention. FIG. 5 is a perspective view showing the sample analysis tool in this example, FIG. 6 is a sectional view taken along an A-A direction in FIG. 5, and FIG. 7 is a sectional view taken along a B-B direction in FIG. 5. In FIGs. 5, 6 and 7, the same portions as those in FIGs. 1, 2 and 3 described above are given the same reference numerals.

As shown in these figures, in this sample analysis tool, the blood cell separation layer has a structure in which a vertical flow filtration separation filter layer 13b is stacked on the lateral flow filtration filter layer 13a. Other than the above, the sample analysis tool of the present example has a structure, a forming material, dimensions and a usage method similar to the sample analysis tool in Example 1 described above.

The vertical flow filtration filter layer 13b is not particularly limited. For example, a glass filter, a polysulfone membrane, a polyethersulfone membrane or the like can be used. Among them, a glass filter and a polyethersulfone membrane are preferable, and a glass filter is more preferable. The vertical flow filtration filter layer 13b may be a commercially available product. The dimensions of the vertical flow filtration filter layer 13b are not particularly limited. In the case where the vertical flow filtration filter layer 13b has a rectangular planar shape, its dimensions are in the range of, for example, 3 to 10 mm long by 3 to 10 mm wide by 0.1 to 0.8 mm thick, preferably 4 to 8 mm long by 4 to 8 mm wide by 0.2 to 0.6 mm thick, and more preferably 5 to 7 mm long by 5 to 7 mm wide by 0.3 to 0.4 mm thick. Although the planar shape of the vertical flow filtration filter layer 13b shown in these figures is rectangular, the present invention is not limited to this. The vertical flow filtration filter layer 13b may have a circular planar shape, for example. As described earlier, in the vertical flow filtration filter layer 13b in the present invention, although a blood cell separation is performed while a blood sample moves principally in the vertical flow direction, there is no particular limitation to this. A part of the blood sample also moves in the lateral flow direction. Furthermore, the vertical flow filtration filter layer 13b may have a single layer structure or a structure in which a plurality of vertical flow filtration filter layers are stacked.

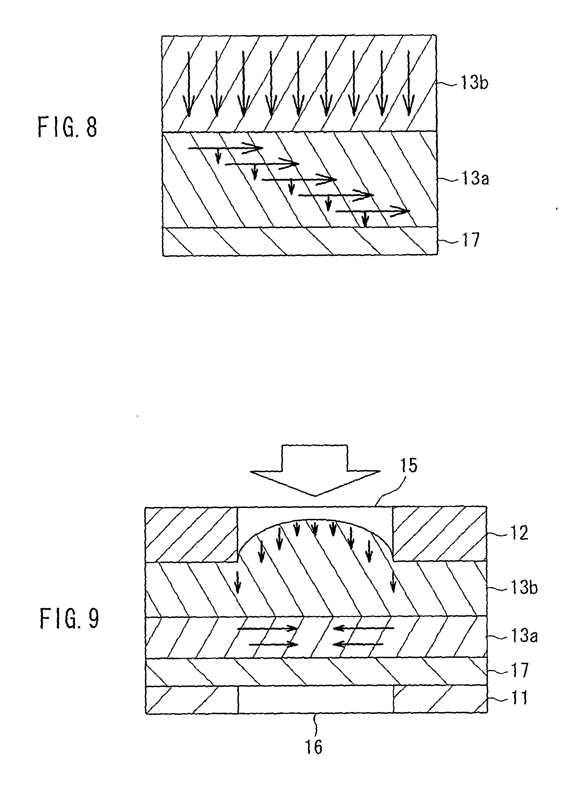

Now, the blood cell separation in this sample analysis tool is carried out as follows, for example. First, a blood sample is prepared similarly to Example 1 and supplied to a blood sample supplying portion through the opening 15 for supplying a blood sample in the cover layer 12. FIG. 8 is a sectional view showing the state of blood cell separation, where the same portions as those in FIGs. 5 to 7 are given the same reference numerals. The supplied blood sample first adheres to the upper surface of the vertical flow filtration filter layer 13b (the blood sample supplying portion) and infiltrates into the above-noted filter layer 13b. Then, as indicated by arrows in FIG. 8, the blood sample moves inside the filter layer 13b in the vertical flow direction. At this time, a blood cell separation is conducted to a certain extent. Subsequently, the blood sample moves to the lateral flow filtration filter layer 13a, where a blood cell separation is conducted while the blood sample is moving in the lateral flow direction. Since the blood sample moves not only in the lateral flow direction but also in the vertical flow direction in the lateral flow filtration filter layer 13a, the plasma obtained by the blood cell separation is introduced into the reagent layer 17, where it reacts with the reagent that is disposed in advance, so that a chromogenic reaction (a color change) or a change in color tone of the reagent occurs.
Then, the chromogenic reaction or the change in color tone of the measuring portion is measured from the opening 16 for measurement in the substrate 11 of the sample analysis tool. This measurement is carried out similarly to Example 1 described above. It should be noted that, although FIG. 8 schematically shows the movement of the blood sample and the arrows along the lateral flow direction indicate the rightward movement of the blood sample, the blood sample of course moves rightward at the same time and also moves downward. Accordingly, in the lateral flow filtration filter in the present invention, the blood sample gradually drops while moving in the lateral flow direction, so that its moving distance can be extended compared with the conventional case.

### Example 3

The following is a description of another example of the sample analysis tool according to the present invention, with reference to FIG. 9.
The sample analysis tool in the present example is similar to that in Example 2 described above except that it is produced by compressing the blood cell separation layer in the sample analysis tool of Example 2. FIG. 9 is a sectional view taken along a B-B direction in FIG. 5, where the same portions as those in FIG. 5 are given the same reference numerals. As shown in the figure, in this sample analysis tool, the vertical flow filtration filter layer 13b and the lateral flow filtration filter layer 13a are compressed by the cover layer 12. A portion corresponding to the opening 15 of the cover layer 12 is left uncompressed, so that a surface portion of the vertical flow filtration filter layer 13b corresponding to the opening 15 bulges like an arc. This compressed state is achieved by using the spacer layers 14 having a thickness smaller than a total thickness (a thickness before compression) of the reagent layer 17 and the two filter layers 13a and 13b and making these spacer layers 14 adhere to both the cover layer 12 and the substrate 11. When such a sample analysis tool is supplied with a blood sample (indicated by a hollow arrow in the figure) through the opening 15, the blood sample infiltrates (moves) quickly in a peripheral portion of the arc-like protruding portion of the vertical flow filtration filter layer 13b and an infiltration speed (a moving speed) becomes slower toward a central portion of the protruding portion as illustrated by black arrows in the figure. As a result, the blood sample is supplied circularly to a portion in the lateral flow filtration filter layer 13a away from a portion corresponding to the center of the measuring portion. Then, in the lateral flow filtration filter layer 13a, most of the blood sample with a partial exception moves toward the central portion. Thus, this sample analysis tool makes it possible to extend the moving distance of the blood sample beyond the distance in the thickness direction and concentrates the plasma obtained by the blood cell separation on a certain position (the central portion in the figure) in the lateral flow filtration filter layer 13a, so that the necessary amount of blood sample can be reduced. The degree to which the filters are compressed is as described earlier.

### Example 4

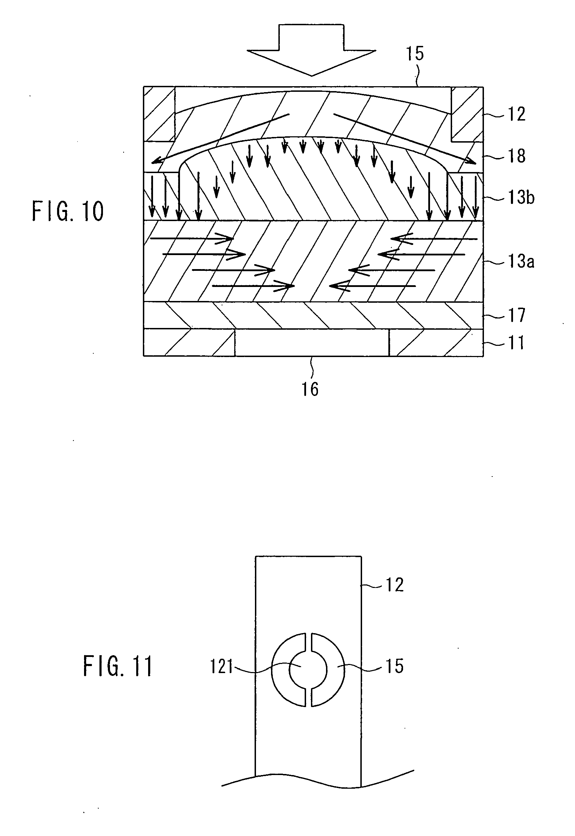

The following is a description of yet another example of the sample analysis tool according to the present invention, with reference to FIG. 10. The sample analysis tool in the present example is similar to those in Examples 2 and 3 described above except that it is produced by disposing a spreading layer 18 on the blood cell separation layer in the sample analysis tool of Example 3 described above and compressing the spreading layer 18, the blood cell separation layer and the reagent layer. FIG. 10 is a sectional view taken along a B-B direction in FIG. 5, where the same portions as those in FIG. 5 are given the same reference numerals. As shown in the figure, in this sample analysis tool, the spreading layer 18, the vertical flow filtration filter layer 13b and the lateral flow filtration filter layer 13a are compressed by the cover layer 12. A portion corresponding to the opening 15 of the cover layer 12 is left uncompressed, so that portions of the spreading layer 18 and the vertical flow filtration filter layer 13b corresponding to the opening 15 bulge like an arc. By disposing the spreading layer 18 as described above, a larger amount of the blood sample is supplied circularly to the periphery of the are-like protruding portion of the vertical flow filtration filter layer 13b and a part immediately below the portion compressed by the cover layer 12. This allows a larger proportion of the blood sample to move over a great distance, thus improving a blood cell separation efficiency and reducing the necessary amount of the blood sample further. The shape of the spreading layer 18 is not particularly limited but may be the same as that of the vertical flow filtration filter layer 13b, for example. Also, the dimensions of the spreading layer 18 are not particularly limited. In the case where the spreading layer 18 has a rectangular planar shape, its dimensions are in the range of, for example, 3 to 10 mm long by 3 to 10 mm wide by 0.03 to 0.35 mm thick, preferably 4 to 8 mm long by 4 to 8 mm wide by 0.03 to 0.3 mm thick, and more preferably 5 to 7 mm long by 5 to 7 mm wide by 0.05 to 0.25 mm thick. As described earlier, the spreading layer 18 can be formed of a mesh, a non-woven fabric, a filter paper or the like, for example. The material of the spreading layer 18 is not particularly limited but can be, for example, polyester, polyethylene, polypropylene, nylon, silk, rayon, cellulose, cotton or the like. Among them, polyester, rayon and nylon are preferable, and polyester and rayon are more preferable.

### Example 5

A sample analysis tool in the present example may have the same configuration as that in Example 1 or 2 except that the opening in the cover layer is formed to be circular. Further, in the sample analysis tool in the present example, the filter layer serving as the blood cell separation layer may be left uncompressed. FIG. 11 is a plan view showing an exemplary shape of the opening for supplying a blood sample in the cover layer. As shown in the figure, two circular-arc slit portions 15 are formed, whereby the opening in the cover layer 12 is made to have a substantially circular shape and a portion surrounded by the two slit portions 15 forms a lid 121. When the blood sample is supplied through this opening, it infiltrates into the blood cell separation layer circularly. As a result, the blood sample is supplied circularly to a portion in the lateral flow filtration filter layer away from the portion corresponding to the center of the measuring portion.

### Example 6

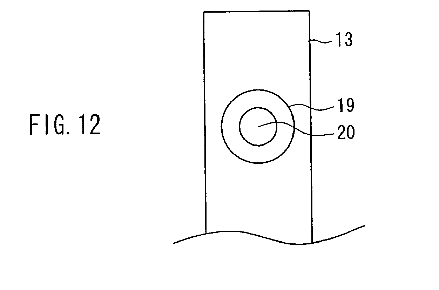

A sample analysis tool in the present example is obtained as follows. In the blood sample supplying portion on the upper surface of the blood cell separation layer, a surface of the blood cell separation layer alone is treated to be water repellent. Other than the above, the sample analysis tool in the present example can be similar to those in all of the examples described above. Further, the sample analysis tool in the present example may have the cover layer or no cover layer. The water repellency is provided to the surface of the blood supplying portion except an end portion thereof. For example, as shown in FIG. 12, in the case where a blood sample supplying portion 19 on the upper surface of the blood cell separation layer has a circular shape, a circular region that is smaller than this circular shape can be formed into a water-repellent portion 20. In this manner, when the blood sample is supplied to the blood sample supplying portion, it infiltrates into the blood cell separation layer circularly. As a result, the blood sample is supplied circularly to a portion in the lateral flow filtration filter layer away from the portion corresponding to the center of the measuring portion. It is preferable that not only the water-repellent portion 20 but also a portion in the upper surface of the blood cell separation layer surrounding the blood sample supplying portion 19 is treated to be water repellent. In the present invention, the treatment for providing water repellency can be carried out using a water repellent. The above-noted water repellent can be, for example, polytetrafluoroethylene, polydimethylsiloxane, polyethylene, polypropylene or the like. Among them, polytetrafluoroethylene and polyethylene are preferable, and polytetrafluoroethylene is more preferable.

### Example 7

In the present example, the blood cell separation was carried out using the sample analysis tool according to Example 4 described above.

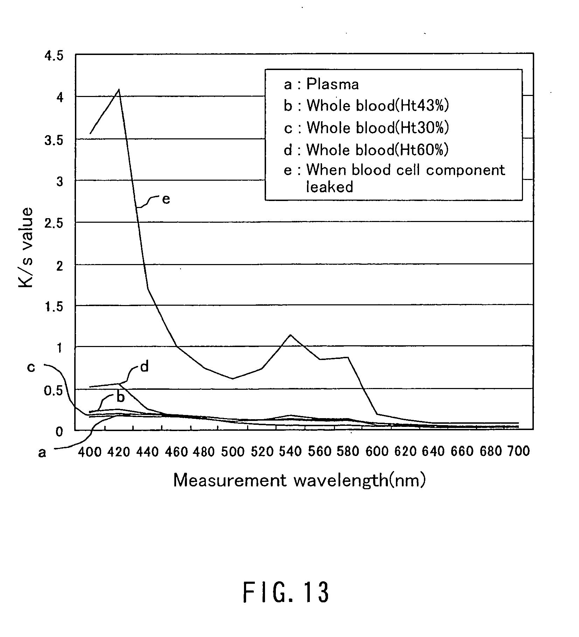

First, a sample analysis tool having a structure shown in FIG. 10 was produced. Members used here and a compression rate were as follows.
Spreading layer: non-woven fabric (trade name R7024; manufactured by TOYOBO CO., LTD.; material: rayon + PET)
Vertical flow filtration filter layer: trade name MF1; manufactured by Whatman plc.; material: glass fiber
Lateral flow filtration filter layer: trade name LF1; manufactured by Whatman plc.; material: glass fiber
Reagent layer (not impregnated with reagent): woven fabric (material: nylon + polystyrene); trade name Biodyne B; manufactured by Pall Corporation; pore diameter: 3.0 µm
Cover layer: white PET film (trade name Lumirror; manufactured by Toray Industries. Inc.); thickness: 188 µm
Substrate: white PET film (trade name Lumirror; manufactured by Toray Industries. Inc.); thickness: 250 µm
Spacer layer: double-coated adhesive tape; product number No. 541; manufactured by Nitto Denko Corporation; thickness: 750 µm
Compression rate: compressed to 40% of original total thickness (before compression) of the above-mentioned two filter layers
Then, 30 µl of each of plasma and blood (whole blood) samples prepared to have a hematocrit (Ht) of 30%, 43% and 60%, respectively, was made to drop and adhere onto the sample analysis tool produced as above. After 120 seconds, the reflectance was measured through the opening for measurement (measuring apparatus: trade name Σ90; manufactured by Nippon Denshoku Industries Co., Ltd.; measurement wavelength: 400 to 700 nm). For comparison, the case in which blood cell components leaked to the measuring surface also was shown. FIG. 13 is a graph showing these results. In this figure, a curve a indicates a spectrum of plasma, a curve b indicates a spectrum of blood with Ht 43%, a curve c indicates a spectrum of blood with Ht 30%, a curve d indicates a spectrum of blood with Ht 60%, and a curve e indicates a spectrum for comparison. As shown in the figure, the sample analysis tool according to the present invention can carry out a blood cell separation with a high separation ability even for blood with a high hematocrit such as the blood with Ht 60%.

### Industrial Applicability

The sample analysis tool according to the present invention can carry out a blood cell separation with a simple structure and at a low cost and allows the blood cell separation with a high separation ability even for blood with a high hematocrit, for example. As a result, a highly accurate and highly reliable analysis is possible. Therefore, the sample analysis tool of the present invention can be used preferably in general blood tests, for example, biochemical tests or clinical tests and has a wide range of uses.

## Claims

1. A sample analysis tool comprising:
a blood cell separation layer;
a reagent; and
a measuring portion;
wherein the blood cell separation layer comprises a lateral flow filtration filter layer,
the measuring portion is located in a lower part of the blood cell separation layer,
a supplied blood sample is subjected to a blood cell separation while moving in the lateral flow filtration filter layer in a lateral flow direction,
the blood sample that has been subjected to the blood cell separation reacts with the reagent, and
a component in the blood sample subjected to the blood cell separation is measured in the measuring portion.

2. The sample analysis tool according to claim 1, comprising a supplying system for supplying the blood sample to a portion in the lateral flow filtration filter layer away from a portion corresponding to a center of the measuring portion.

3. The sample analysis tool according to claim 1, further comprising
a substrate, and
a reagent layer,
wherein the reagent layer comprises the reagent,
the reagent layer is disposed on the substrate,
the blood cell separation layer is stacked on the reagent layer,
a part or a whole of an upper surface of the blood cell separation layer serves as a blood sample supplying portion,
a part or a whole of a lower surface of the reagent layer serves as the measuring portion,
the blood sample supplied from the supplying portion is subjected to the blood cell separation by the blood cell separation layer,
the blood sample that has been subjected to the blood cell separation is introduced into the reagent layer and reacts with the reagent, and
the component in the blood sample subjected to the blood cell separation is measured in the measuring portion.

4. The sample analysis tool according to claim 1, wherein the blood cell separation layer further comprises a vertical flow filtration filter layer,
the vertical flow filtration filter layer is stacked on the lateral flow filtration filter layer, and
the supplied blood sample first is subjected to a blood cell separation while moving in the vertical flow filtration filter layer in a vertical flow direction and then subjected to the blood cell separation while moving in the lateral flow filtration filter layer in the lateral flow direction.

5. The sample analysis tool according to claim 1, further comprising a cover layer disposed on the blood cell separation layer,
wherein the cover layer has an opening for supplying the blood sample to the blood cell separation layer, and
a portion of an upper surface of the blood cell separation layer corresponding to the opening serves as a blood sample supplying portion.

6. The sample analysis tool according to claim 5, further comprising a spacer layer disposed on the substrate,
wherein the cover layer is disposed on the spacer layer and the blood cell separation layer.

7. The sample analysis tool according to claim 6, wherein the spacer layer adheres to both the substrate and the cover layer and has a thickness designed to be smaller than a pre-compressed thickness of the blood cell separation layer, whereby the blood cell separation layer is compressed by the cover layer except the opening.

8. The sample analysis tool according to claim 7, wherein a degree to which the blood cell separation layer is compressed is such that a compressed thickness is at least 30 and smaller than 100, where the pre-compressed thickness of the blood cell separation layer is expressed by 100.

9. The sample analysis tool according to claim 7, further comprising a spreading layer stacked on the blood cell separation layer.

10. The sample analysis tool according to claim 3, wherein the substrate has an opening for measurement at a position matched with the measuring portion of the reagent layer.

11. The sample analysis tool according to claim 10, wherein an opening in a cover layer has a larger diameter than the opening for measurement in the substrate.

12. The sample analysis tool according to claim 3, wherein the blood sample supplying portion on the upper surface of the blood cell separation layer except an end portion thereof is treated to be water repellent.

13. The sample analysis tool according to claim 5, wherein the opening in the cover layer is substantially circular.

14. The sample analysis tool according to claim 4, wherein the vertical flow filtration filter layer and the lateral flow filtration filter layer are formed of a glass filter.

15. The sample analysis tool according to claim 9, wherein the spreading layer is formed of at least one selected from the group consisting of a mesh, a non-woven fabric and a filter paper.

16. The sample analysis tool according to claim 1, wherein an item to be tested is at least one selected from the group consisting of glucose, cholesterol, triglyceride, ammonia, a uric acid, creatinine, urea nitrogen, calcium, inorganic phosphorus, magnesium, GGT, GOT, GPT, LDH, amylase, a total protein amount, albumin, fructosamine, creatine phosphokinase, bilirubin, alkaline phosphatase and HDL.
